# EUROPEAN PATENT SPECIFICATION

(11) **EP 2 091 993 B1**
(45) Date of publication and mention of the grant of the patent: **11.05.2011**
(21) Application number: 07846803.0
(22) Date of filing: 24.11.2007
(51) Int. Cl.: C08G 18/79, C07C 267/00

(54) **PROCESS FOR PREPARING MODIFIED POLYISOCYANATE**
VERFAHREN ZUR HERSTELLUNG VON MODIFIZIERTEM POLYISOCYANAT
PROCÉDÉ DE PRÉPARATION DE POLYISOCYANATE MODIFIÉ

(30) Priority: 07.12.2006 JP 2006330240
(43) Date of publication of application: 26.08.2009
(73) Proprietor: Bayer MaterialScience AG, 51368 Leverkusen (DE)
(72) Inventor: KAMIYAMA, Nobuhiro, Itami-City Hyogo 664-0874 (JP); FUKUBA, Tomoaki, Niihama-City Ehime 792-0060 (JP); MANO, Takashi, Niihama-City Ehime 792-0025 (JP)
(86) International application number: PCT/EP2007/010226
(87) International publication number: WO 2008/067920

(56) References cited:
- EP-A- 1 671 988
- US-A- 4 014 935

## Description

### Technical Field

The present invention relates to a process for preparing a modified polyisocyanate by carbodiimidizing an organic polyisocyanate in the presence of a phospholine-based catalyst.

### Background of the Invention

The conversion of the isocyanate groups of an organic polyisocyanate to give carbodiimide groups (hereinafter referred to also as "CD") has hitherto been carried out by heating the organic polyisocyanate at a high temperature.To prevent the polymerization of the organic polyisocyanate at such a high temperature and to reduce a consumed energy in the above-mentioned carbodiimidization, the use of a catalyst which allows carbodiimidization at a relatively low temperature is proposed. Particularly, a phospholine-based catalyst, especially a phospholine oxide-based catalyst, is preferable, since such a catalyst shows a higher catalytic activity even under a mild temperature condition and initiates and accelerates the carbodiimidization reaction, and this matter is disclosed in US Patent No. 2853473, EP-A-515933 and US Patent No. 6120699.

However, the carbodiimidization reaction is known to be accompanied by formation of carbon dioxide. To terminate the carbodiimidization reaction, it is needed to inactivate the phospholine-based catalyst, especially the phospholine oxide-based catalyst and remove it. If such inactivation and removal are not conducted, the carbodiimidization reaction continues, and the formation of carbon dioxide (hereinafter referred to as "release of a gas) also continues. This tendency is also found during the storage of an organic polyisocyanate having been subjected to carbodiimidization, for example, when the inactivation or removal of the catalyst is insufficient. In such a case, a gas is vigorously released particularly at a high temperature. As a result, the internal pressure of a container (e.g. a tank, a drum or a petroleum can) which receives the carbodiimidized organic polyisocyanate increases to expand the container, or sometimes burst it.

EP-A-515933 and US Patent No. 6120699 disclose the use of, for example, a higher molar equivalent of an acid, an acid chloride, chloroformate, silylated acid or trimethylylsilyltrifluoromethane sulfonate as a terminator for the carbodiimidization reaction in the presence of the phospholine-based catalyst or the phospholine oxide-based catalyst. This method has a problem in that addition of a large amount of the terminator is needed, since addition of a small amount of the terminator is insufficient in the effect to terminate the carbodiimidization reaction by way of the inactivation of the catalyst. However, addition of a large amount of, for example, a silylated acid is undesirable, because the resultant isocyanate having a carbodiimide group and/or an uretonimine group tends to have a considerably large color number, and because a prepolymer obtained from such an isocyanate is also colored. Thus, the use of such a colored prepolymer is limited.

JP-B-57-36906/1982 discloses a method for obtaining an organic isocyanate excellent in heat stability by using an adsorbent to adsorb a catalyst, thereby inactivating and removing the catalyst. This publication discloses the use of siliceous earth, adsorption carbon and a metal oxide as the adsorbent, but does not describe the particulars of the respective adsorbents. The siliceous earth, although given as a preferable adsorbent, is low in content of silicic anhydride which is excellent in adsorbing effect. In addition, the adsorbent is likely to contain a relatively large moisture, and therefore, there is a possible problem in the control of reaction between an organic isocyanate and the moisture of the adsorbent. Thus, the use of such an adsorbent is unsatisfactory.
Patent Literature 1: US Patent No. 2853473
Patent Literature 2: EP-A-515933
Patent Literature 3: US Patent No. 6120699
Patent Literature 4: JP-A-51-122023/1976
Patent Literature 5: JP-B-57-36906/1982

Under such a circumstance, there grows a demand for a terminator capable of inactivating a phospholine-based catalyst, especially a phospholine oxide-based catalyst, thereby terminating a carbodiimidization reaction and facilitating the removal of the catalyst, and further decreasing the color number of the resultant polyisocyanate.

### Disclosure of the Invention

### Problems to be solved by the Invention

An object of the present invention is to provide a CD (carbodiimidization) modified polyisocyanate having a smaller color number and excellent heat stability.

### Means for solving the problems

As a result of the present inventors' intensive investigation for solving the above-discussed problems, they have found that a CD modified polyisocyanate having a smaller color number and excellent heat stability can be obtained, and have accomplished the present invention.

The present invention provides a process for preparing a modified polyisocyanate by carbodiimidizing an organic polyisocyanate in the presence of a phospholine-based catalyst, characterized in that silicon dioxide particles having a specific surface area of at least 400 m²/g and an oil adsorption of at least 180 mL/100 g are used to adsorb and remove the phospholine-based catalyst to thereby terminate the carbodiimidization reaction.

The present invention also provides a modified polyisocyanate obtained by the above-mentioned process.

The present invention further provides a mixture of the above-mentioned modified polyisocyanate with a non-carbodiimidized organic polyisocyanate, and a prepolymer having a terminal isocyanate group, obtained from the above-mentioned modified polyisocyanate or the above-mentioned mixture of the modified polyisocyanate.

### Effect of the Invention

The CD modified polyisocyanate obtained by the present invention shows a smaller color number in production, and is excellent in heat stability and thus does not release a gas at a temperature of 45°C or lower which is a general storage temperature. Even if the storage temperature rises to about 80°C, this CD modified polyisocyanate does not release a gas. Also, a prepolymer having a terminal isocyanate group (hereinafter referred to as "a prepolymer having a terminal NCO"), prepared by using a mixture of the CD modified polyisocyanate with a non-carbodiimidized organic polyisocyanate, does not release a gas during and after the preparation thereof, and also during the storage thereof, because of the excellent heat stability.

### Best Modes for Carrying out the Invention

Hereinafter, the present invention will be described in detail.

In the process of the present invention, the carbodiimidization (CD) reaction is carried out in the presence of at least one phospholine-based catalyst.

The phospholine-based catalyst is preferably a phospholine oxide. Examples of the phospholine-based catalyst include 1-methylphospholine-1-oxide and 1-phenyl-3-methylphospholine-1-oxide. Among them, 1-methyphospholine-1-oxide is preferred.

The amount of the phospholine-based catalyst to be used in the present invention varies depending on the quality of an organic polyisocyanate. Accordingly, a required amount of the phospholine-based catalyst can be determined by a simple preliminary test. In the present invention, the amount (by weight) of the phospholine-based catalyst is preferably from 0.5 to 20 ppm, particularly from 1 to 10 ppm, based on the weight of the organic polyisocyanate.

The carbodiimidization reaction is carried out within a temperature range of, generally, from 50 to 150°C, desirably from 70 to 90°C. This temperature also can be determined by a simple preliminary test. The carbodiimidization reaction time, namely, a time interval from a point of time at which the carbodiimidization reaction is initiated to a point of time at which an adsorbent is added, is preferably from 1.0 to 20 hours, for example, from 2.0 to 10 hours.

Desirably, the carbodiimidization reaction is terminated when the degree of carbodiimidization has reached generally 3 to 50%, preferably 5 to 30%. The degree of carbodiimidization is expressed by a ratio of the carbodiimidized isocyanate groups to a total of the isocyanate groups (hereinafter referred to as "NCO groups") in the organic polyisocyanate.

The degree of carbodimidization can be determined by measuring the NCO content while the carbodiimidization reaction is proceeding. The NCO content in the CD modified polyisocyanate can be readily determined by a known titration or an appropriate on-line analysis.

The degree of carbodimidization also can be determined from the amount of carbon dioxide which is released from the organic polyisocyanate while the carbodiimidization reaction is proceeding. The volume of carbon dioxide can be easily measured, and the NCO content can be easily determined by a conversion formula of the volume of carbon dioxide.

[In the present invention, the phospholine-based catalyst is adsorbed by the adsorbent so as to be inactivated, and then is removed.

In the present invention, silicon dioxide particles are used as the adsorbent.

The specific surface area of the silicon dioxide particles is preferably from 400 to 800 m²/g, particularly from 500 to 750 m²/g.

The specific surface area can be measured according to the silica gel test method regulated in JIS K1150.

The oil absorption of the silicon dioxide particles is preferably from 180 to 500 ml/100 g, particularly from 250 to 500 ml/100 g, more preferably from 350 to 450 ml/100 g.

The oil absorption can be measured according to the pigment test method regulated in JIS K5101.

The use of silicon dioxide particles which satisfy both of a specific surface area of from 500 to 750 m²/g and an oil absorption of from 350 to 450 mL/100 g is still more preferable, since such silicon dioxide particles provide a higher effect to adsorb and inactivate the phospholilne-based catalyst to thereby terminate the carbodiimidization reaction.

Lower moisture content of the silicon dioxide particles is preferable. Higher purity of the silicon dioxide is preferable.

The moisture content of the silicon dioxide particles is preferably 20 wt.% or less, more preferably 5 wt.% or less, particularly 3 wt.% or less.

The purity of silicon dioxide of the silicon dioxide particles is preferably 80 wt.% or more, more preferably 95 wt.% or more.

The average particle size of the silicon dioxide particles is preferably from 0.5 to 25.0 micrometers, more preferably from 1.0 to 10.0 micrometers, still more preferably from 4.0 to 5.5 micrometers. The silicon dioxide particles having an average particle size of from 0.5 to 25.0 micrometers are easily removed from the CD modified polyisocyanate, and further can sufficiently adsorb the phospholine-based catalyst in the CD modified polyisocyanate to sufficiently terminate the carbodiimidization reaction.

The shapes of the silicon dioxide particles may be spherical or irregular. The spherical silicon dioxide particles are preferred, since such particles are hard to be ground when added to the modified polyisocyanate and stirred and mixed therewith. Preferably, the silicon dioxide particles are porous, because the porous silicon dioxide particles can adsorb more phospholine-based catalyst than nonporous silicon dioxide particles. The silicon dioxide particles are preferably solid rather than hollow. Preferably, the silicon dioxide particles are not of fumed silica.

Examples of commercially available silicon dioxide particles are God Balls E-2C, B-6C, B-25C, AF-16C and SF-16C manufactured by Suzuki Yushi Industrial Co., Ltd.

The silicon dioxide particles are used in an amount of preferably from 2.5 to 2,000 wt. parts, particularly from 5 to 1,000 wt. parts, per 1 wt. part of the phospholine-based catalyst.

The use of 2.5 to 2,000 wt. parts of the silicon dioxide particles per 1 wt. part of the phospholine-based catalyst is sufficient to adsorb and inactivate the phospholine-based catalyst, and facilitates the separation and removal of the modified polyisocyanate by way of decantation, filtration or centrifugal separation of the catalyst and the adsorbed material of the absorbent.

The inactivation of the catalyst by the use of the adsorbent is generally carried out by adding the adsorbent to the reaction mixture and stirring them for 0.5 to 1 hour. While this inactivation may be carried out at an arbitrary temperature between a room temperature and the carbodiimidization temperature, a temperature of from 40 to 80°C is preferred for the inactivation, in view of reduction of time and energy efficiency.

The silicon dioxide particles as used in the present invention are excellent in adsorption capacity to the catalyst. Therefore, the use of such silicon dioxide particles is effective to facilitate the separation and removal of the silicon dioxide particles having adsorbed the catalyst by filtration, and to save the adsorption time as compared with the conventional method to thereby lower the heat history. Accordingly, the production steps can be decreased, which leads to a lower cost. Moreover, the color number of the resultant CD modified polyisocyanate is not increased, as compared with that of a modified polyisocyanate obtained using various acids or silylated acids as the terminator.

For example, the following are used as the organic polyisocyanate to be used in the present invention: that is, aromatic diisocyanates such as 2,4- and 2,6-toluene diisocyanate (TDI) and a mixture thereof, 4,4'-diphenylmethane diisocyanate (4,4' MDI) and isomers thereof, i.e., 2,2'-diphenylmethane diisocyanate (2,2' MDI) and 2,4'-diphenylmethane diisocyanate (2,4' MDI), and mixtures thereof.

Further, polymethylene polyphenylmethane polyisocyanate (hereinafter referred to as "a polymeric MDI") which contains a tri- or higher-functional polyisocyanate is also used as the organic polyisocyanate.

Preferable is an aromatic diisocyanate obtained by mixing 4,4' MDI and isomers thereof in an arbitrary ratio.

In the aromatic diisocyanate comprising 4,4' MDI and isomers thereof, an example of the ratio of the isomers is as follows: 0 to 100 wt.% (for example, 1 to 99 wt.%) of 4,4' MDI, 0 to 65 wt.% (for example, 0.1 to 50 wt.%) of 2,4' MDI, and 0 to 8 wt.% (for example, 0.1 to 5 wt.%) of 2,2' MDI, provided that the total of the isomers is 100%.

Preferably, the organic polyisocyanate before the carbodiimidization reaction has a NCO content of 25.0 to 38.0 wt.%, particularly 28.0 to 36.0 wt.%.

The modified polyisocyanate of the present invention, obtained by converting the NCO group of the aromatic diisocyanate to a CD group, is a pale color liquid which shows a smaller color number at a room temperature. The APHA value indicating the color number is generally 40 or less, for example, 20 or less. A mixture of the modified polyisocyanate with the polymeric MDI can be in the form of a liquid at a temperature of as low as, for example, 0°C, depending on the mixing ratio of the polymeric MDI. When the modified polyisocyanate is mixed with other organic polyisocyanate which is not carbodiimidized, the resulting mixture can be in the form of a liquid even at a temperature lower than 0° C.

Generally, in the CD modified polyisocyanate, the NCO groups which are not converted to the CD groups can be reacted with the CD groups at a normal temperature (for example, 25° C) or lower after the carbodiimidization, to thereby form uretonimine groups (hereinafter referred to as "UI"). At this reaction, all the CD groups are not converted into UI groups, and thus, some of them are left to be CD groups. Thus, the resultant polyisocyanate contains CD groups and UI groups.

In a mixture of the CD modified polyisocyanate with an organic polyisocyanate which is not carbodiimidized, the UI groups are easily formed.

The weight ratio of the CD modified polyisocyanate to the organic polyisocyanate which is not carbodiimidized is from 1:99 to 99:1, preferably from 10:90 to 90:10.

This polyisocyanate containing CD groups and UI groups is suitable for use in manufacturing of polyurethane resin products, and is particularly suitable for use in manufacturing of polyurethane elastomers and non-foamed or high density integral skin polyurethane foams which are not required to be foamed at a low density and which are used for interior and exterior materials for automobile components, shoe soles and furniture.

When the UI groups are formed under the condition where the inactivation of the phospholine-based catalyst is insufficient or where traces of the catalyst is left to remain because of the insufficient filtration removal of the catalyst, the carbodimidization reaction proceeds. As a result, a desired polyisocyanate containing CD groups and UI groups can not be obtained, and also, a gas is released to increase the internal pressure of the storage container. This event is also caused in the mixture of the CD modified polyisocyanate with the organic polyisocyanate which is not carbodiimidized.

Further, also in the production of a prepolymer having a terminal NCO group, by reacting the polyisocyanate containing CD groups and/or UI groups with a polyol or the like generally used in polyurethane, the carbodiimidization reaction is concurrently caused because of an increase in reaction temperature for obtaining the prepolymer having the terminal NCO group, if the inactivation of the phospholine-based catalyst or the removal thereof by filtration is insufficient. As a result, an NCO group-terminated prepolymer having the desired properties can not be obtained, and also, a gas is released to dangerously cause an abnormal increase in internal pressure during the reaction.

Moreover, during the storage of such a prepolymer having a terminal NCO group, a gas is released with an increase in storage temperature, which leads to an increase in the internal pressure of the storage container.

When polyurethane resin products such as polyurethane elastomers and non-foamed or high density integral skin polyurethane foams are manufactured from the polyisocyanate containing CD groups and/or UI groups, or the prepolymer having a terminal NCO group, prepared from the same polyisocyanate, exothermic heat accompanying the reaction for forming the polyurethane resin causes a gas release to foam the polyurethane resin more than needed, so that resultant polyurethane resin becomes lower in density. Thus, polyurethane resin products satisfactory in performance, etc. can not be obtained.

Preferably, the CD modified polyisocyanate of the present invention is prepared by carbodiimidizing the organic polyisocyanate in the presence of 0.5 to 20 ppm of the phospholine-based catalyst based on the weight of the organic polyisocyanate, on condition that specified silicon dioxide particles having a specific surface area of 400 to 800 m²/g and an oil absorption of 180 to 500 mL/100 g are used in an amount of 2.5 to 2,000 wt. parts per 1 wt. part of the phospholine-based catalyst so as to adsorb and inactivate the phospholine-based catalyst, and that the phospholine-based catalyst is then removed by filtration separation of the particles to thereby terminate the carbodiimidization reaction.

The thus obtained CD modified polyisocyanate has a smaller color number and excellent heat stability, and therefore is excellent in storage stability.

Also, when UI groups are formed in the CD modified polyisocyanate at a normal temperature (for example 25°C), there can be obtained a modified polyisocyanate containing CD groups and/or UI groups, suitable for use in manufacturing of a polyurethane elastomer or a non-foamed or high density integral skin polyurethane foam, because the CD modified polyisocyanate is excellent in heat stability and thus is not further carbodiimidized.

Furthermore, any gas release attributed to the proceeding of the carbodiimidization is not caused. Still furthermore, the prepolymer having a terminal NCO group, obtained from the polyisocyanate containing CD groups and/or UI groups, is also excellent in not only heat stability during the preparation thereof but also storage stability after the preparation thereof, and further has a smaller color number.

The present invention will be described in more detail by the following Examples, in which "part" and "%" means "wt. part" and "wt.%", unless otherwise specified.

The color number of the CD modified polyisocyanate, the degree of hardness in filtration of the adsorbent containing the absorbed catalyst, and the heat stability (or storage stability) of the CD modified polyisocyanate were evaluated as follows.

### (1) Color number

When the APHA value (specified by the color number testing method according to JIS K4101-1993) is 40 or less, the corresponding color number is small and satisfactory.

### (2) Degree of hardness in filtration of the adsorbent containing the adsorbed catalyst and the like

The degree of hardness in filtration is determined based on a time spent for the filtration of 1 kg of the CD modified polyisocyanate, using a filter paper having an effective trapped particle size of 3 microns. When this time is 60 minutes or longer, the filtration of the adsorbent is evaluated to be hard.

### (3) Heat stability (or storage stability)

The CD modified polyisocyanate (400 g) was charged in a 500-ml petroleum drum and is left to stand at 45°C for 24 hours. Thereafter, the heat stability of the CD modified polyisocyanate is evaluated based on the level of expansion of the petroleum drum (levels 0 to 6):

| | |
|---|---|
| Level 0 (good): | No expansion was observed in the drum. |
| | |
| Level 1 (good): | Slight expansion was observed in the side wall of the drum, and slight resistance to depression was felt when the drum was pushed by one's hand. |
| Level 2 (slightly bad): | Expansion was observed in the side wall of the drum, and resistance to depression was felt when the drum was pushed by one's hand. |
| | |
| Level 3 (bad): | Large expansion was observed in the side wall of the drum, and the drum was not depressed when pushed by one's hand. |
| | |
| Level 4 (bad): | Large expansion was observed in the side wall of the drum, and expansion was observed also in a half of the top plate of the drum. |
| | |
| Level 5 (bad): | Large expansion was observed in the side wall of the drum, and expansion was observed also in a whole of the top plate of the drum. |
| | |
| Level 6 (bad): | The original shape of the drum was not left to remain. |

### Example 1

### Production of Polyisocyanate Containing CD Group and/or UI Group

(1) To 1,000 parts of 4,4'-MDI (having a NCO content of 33.6%), 3.5 ppm of 1-methylphospholine-1-oxide (a 30% toluene solution, an effective catalyst amount of 1.05 ppm) as a catalyst for a carbodiimidization reaction was added, and the resulting mixture was heated at 80°C for about 6 hours for the carbodiimidization reaction. After the resultant CD modified polyisocyanate was cooled to 60°C, the concentration of isocyanate groups (NCO content) was 29.6% (The degree of carbodiimidization was 11 %).
(2) To the CD modified polyisocyanate obtained in the step (1) were immediately added 0.05% of silicon dioxide particles (having a purity of 85%, a specific surface area of 650 m²/g, an oil absorption of 350 mL/100 g and an average particle diameter of 4.5 micrometers) as an adsorbent, which was 476 times as the amount of 1-methylphospholine-1-oxide, and the NCO content was measured at every about 30 minute intervals under stirring. The NCO content was found to be 29.6% after one hour had passed, and thus, little change was observed in the NCO content. Thereafter, stirring was continued for about 4 hours, and the NCO content was checked. The NCO content was 29.4%, and was found to be hardly lowered.
(3) The silicon dioxide particles having adsorbed the catalyst added in the step (2) were filtered through a filter paper having an effective trapped particle diameter of 3 microns. The filtration was smoothly carried out in 50 minutes without any clogging.
   The CD modified polyisocyanate had a color number of as small as 30 APHA, and it was a clear and colorless liquid having a NCO content of 29.4%. The viscosity of the liquid at 25°C was 37 mPas.
(4) To further examine the heat stability (or the storage stability), 400 g of the CD modified polyisocyanate obtained in the step (3) was charged in a 500-mL petroleum drum, of which the internal atmosphere was displaced with a nitrogen gas and was then sealed. Then, the CD modified polyisocyanate in the drum was left to stand at 45°C for 24 hours (to form UI groups). The expansion of the petroleum drum was at level 1, from which it was evaluated that a gas was hardly released.

### Example 2

### Production of Modified Polyisocyanate Containing CD Group and/or UI Group

(1) Example 1 was repeated, except that the heating time was changed to about 5.1 hours. The NCO content of the CD modified polyisocyanate, found after cooling to 60° C, was 29.6% (the degree of carbodiimidization was 11 %).
(2) To the CD modified polyisocyanate obtained in the step (1) were immediately added 0.01% of silicon dioxide particles (having a purity of 85%, a specific surface area of 650 m²/g, an oil absorption of 350 ml/100 g and an average particle diameter of 4.5 micrometers) as an absorbent, which was 95 times as the amount of 1-methylphospholine-1-oxide as the catalyst. The NCO content of the CD modified polyisocyanate was measured at every about 30 minutes intervals under stirring. The NCO content became constant at 29.4% after one hour had passed, and little decrease was observed in NCO content. Thereafter, stirring was continued for about 4 hours for confirmation, and the NCO content was examined. As a result, it was found that the NCO content was 29.1 %, and was hardly lowered.
(3) The silicon dioxide particles having adsorbed the catalyst added in the step (2) were filtered through a filter paper having an effective trapped particle diameter of 3 microns. The filtration was smoothly done in so short a time as 9 minutes without any clogging.
   This CD modified polyisocyanate had a color number of as small as 30 APHA, and it was a clear and colorless liquid having a NCO content of 29.0%. The viscosity of the CD modified polyisocyanate at 25°C was 47 mPas.
(4) To further examine the heat stability (or the storage stability), 400 g of the CD modified polyisocyanate obtained in the step (3) was charged in a 500-ml petroleum drum, of which the internal atmosphere was displaced with a nitrogen gas and was then sealed. The CD modified polyisocyanate in the drum was left to stand at 45° C for 24 hours (to form UI groups). The expansion of the drum was at level 1, from which it was found that a gas was hardly released.

### Comparative Example 1

### Production of Polyisocyanate Containing CD Group and/or UI Group

(1) A carbodiimidization reaction was carried out in the presence of 3.0 ppm of 1-methylphospholine-1-oxide (a 30% toluene solution, an effective catalyst amount of 0.9 ppm) as a catalyst, by heating at a temperature of 83°C for about 7.6 hours. The NCO content of the CD modified polyisocyanate found after cooling to 60° C was 29.5% (the degree of carbodiimidization was 11%).
(2) To the CD modified polyisocyanate obtained in the step (1) was immediately added 10 ppm of trimethylsilyltrifluoromethane sulfonate (TMST) as a terminator for the carbodiimidization reaction, which was 11 times as the amount of 1-methylphospholine-1-oxide. The NCO content of the CD modified polyisocyanate was measured at every about 30 minute intervals under stirring. The NCO content became 29.3% after one hour had passed, and then, the NCO content was examined after 4 hours had passed. The NCO content was found to be further lowered to 28.9%, and thereafter, the NCO content became constant at 28.9%. The CD modified polyisocyanate had a color number of as large as 80 APHA, and thus, it was colored. The viscosity of the CD modified polyisocyanate at 25° C was 47 mPas.
(3) To examine the heat stability (or the storage stability), 400 g of the CD modified polyisocyanate obtained in the step (2) was charged in a 500-ml petroleum drum, of which the internal atmosphere was displaced with a nitrogen gas and was then sealed. The CD modified polyisocyanate in the drum was left to stand at 45°C for 24 hours (to form UI groups). The expansion of the drum was at level 2, from which it was evaluated that a gas was released.

### Comparative Example 2

### Production of Polyisocyanate Containing CD Group and/or UI Group

(1) A carbodiimidization reaction was carried out in the presence of 3.6 ppm of 1-methylphospholine-1-oxide as a catalyst (a 30% toluene solution, an effective catalyst amount of 1.08 ppm) by heating at a temperature of 78°C for about 7.5 hours. The NCO content of the CD modified polyisocyanate found after cooling to 60°C was 29.6% (the degree of carbodiimidization was 11 %).
(2) To the CD modified polyisocyanate obtained in the step (1) was immediately added 0.05% of aluminum oxide as an terminator for the carbodiimidization reaction, which was 463 times as the amount of 1-methylphospholine-1-oxide. The NCO content of the CD modified polyisocyanate was measured at every about 30 minute intervals under stirring. The NCO content was found to be 29.4% after one hour had passed, and then, the NCO content was examined after 4 hours had passed. The NCO content was found to be lowered to 29.0%.
(3) The aluminum oxide having adsorbed the catalyst added in the step (2) was filtered through a filter paper having an effective trapped particle diameter of 3 microns. The filtration was smoothly carried out in so short a time as 5 minutes without any clogging.
   This CD modified polyisocyanate had a color number of as small as 30 APHA, and the viscosity thereof at 25°C was 47 mPas. The final NCO content was lowered to 28.7%.
(4) To examine the heat stability (or the storage stability), 400 g of the CD modified polyisocyanate obtained in the step (3) was charged in a 500-mL petroleum drum, of which the internal atmosphere was displaced with a nitrogen gas and was then sealed. The CD modified polyisocyanate was left to stand at 45°C for 24 hours (to form UI groups). The expansion of the drum was at level 3, from which it was evaluated that a gas was released.

### Comparative Example 3

### Production of Polyisocyanate Containing CD Group and/or UI Group

(1) Example 1 was repeated, except that the heating time was changed to about 6.5 hours. The NCO content of the CD modified polyisocyanate found after cooling to 60°C was 29.9% (the degree of carbodiimidization was 11 %).
(2) The NCO content of the CD modified polyisocyanate was measured at every about 30 minute intervals under stirring, without using any terminator for inactivating the catalyst. The NCO content was found to be 29.5% after one hour had passed, and the NCO content was examined after 4 hours had passed. The NCO content was found to be lowered to 28.7%, and thereafter, the NCO content was further lowered to 28.5%
(3) To examine the heat stability (or the storage stability), 400 g of the CD modified polyisocyanate obtained in the step (2) was charged in a 500-mL petroleum drum, of which the internal atmosphere was displaced with a nitrogen gas and was then sealed. The CD modified polyisocyanate in the drum was left to stand at 45°C for 24 hours (to form UI groups). The expansion of the drum was at level 4, from which it was evaluated that a gas was largely released.

### Comparative Example 4

(1) A carbodiimidization reaction was carried out in the same manner as in Example 1, except that the heating temperature was changed to 83°C, and the heating time, to about 7.3 hours. The NCO content of the CD modified polyisocyanate found after cooling to 60°C was 29.3% (the degree of carbodiimidization was 11%).
(2) To the CD modified polyisocyanate obtained in the step (1) were immediately added 0.01 % of silicon dioxide particles (having a specific surface area of 300 m²/g, an oil absorption of 105 mL/100 g and an average particle diameter of 4.0 micrometers) as an adsorbent which was 95 times as the amount of 1-methylphospholine-1-oxide as the catalyst. The NCO content of the CD modified polyisocyanate was measured at every about 30 minute intervals under stirring. The NCO content was found to be 29.1 % after one hour had passed, and the NCO content was lowered to 28.5% after 4 hours had passed.
(3) The silicon dioxide particles having adsorbed the catalyst added in the step (2) were filtered through a filter paper of an effective trapped particle diameter of 3 microns. The filtration was smoothly carried out in so short a time as 3 minutes without any clogging.
   The CD modified polyisocyanate had a color number of as small as 30 APHA, and the viscosity thereof at 25°C was 81 mPas, and the final NCO content was lowered to 27.7%.
(4) To examine the heat stability (or the storage stability), 400 g of the CD modified polyisocyanate obtained in the step (3) was charged in a 500-ml petroleum drum, of which the internal atmosphere was displaced with a nitrogen gas and was then sealed. The CD modified polyisocyanate in the drum was left to stand at 45°C for 24 hours (to form UI groups). The expansion of the drum was at level 3, from which it was evaluated that a gas was released.

### Comparative Example 5

(1) A carbodiimidization reaction was carried out in the same manner as in Example 1, except that the heating temperature was changed to 83°C, and the heating time, to about 6.3 hours. The NCO content of the CD modified polyisocyanate found after cooling to 60°C was 29.5% (the degree of carbodiimidization was 11 %).
(2) To the CD modified polyisocyanate obtained in the step (1) were immediately added 0.01 % of silicon dioxide particles (having a specific surface area of 350 m²/g, an oil absorption of 160 ml/100 g and an average particle diameter of 0.9 micrometers) as an adsorbent, which was 95 times as the amount of 1-methylphospholine-1-oxide as the catalyst. The NCO content of the CD modified polyisocyanate was measured at every about 30 minute intervals under stirring. The NCO content was 29.3% after one hour had passed, and the NCO content was lowered to 28.8% after 4 hours had passed.
(3) The silicon dioxide particles having adsorbed the catalyst added in the step (2) were filtered through a filter paper having an effective trapped particle diameter of 3 microns. The NCO content found after the filtration was 28.7%; the color number of the CD modified polyisocyanate was as small as 30 APHA; and the viscosity thereof at 25°C was 81 mPas. However, the filtration was not smoothly carried out, since the filtration time was so long as 96 minutes.
(4) To examine the heat stability (or the storage stability), 400 g of the CD modified polyisocyanate obtained in the step (3) was charged in a 500-ml petroleum drum, of which the internal atmosphere was displaced with a nitrogen gas and was then sealed. The CD modified polyisocyanate in the drum was left to stand at 45°C for 24 hours (to form UI groups). The expansion of the drum was at level 2, from which it was evaluated that a gas was released.

**Table 1**

| | Ex. 1 | Ex.2 | C.Ex.1 | C.Ex.2 | C.Ex.3 | C.Ex.4 | C.Ex.5 |
|---|---|---|---|---|---|---|---|
| (1) 4,4'MDI (parts) | 1,000 | ← | ← | ← | ← | ← | ← |
| 1-Methylphospholine-1-oxide (ppm) (a 30% toluene solution) | 3.5 | ← | 3.0 | 3.6 | 3.5 | ← | ← |
| NCO content (%) | 29.6 | 29.6 | 29.5 | 29.6 | 29.9 | 29.3 | 29.5 |
| (2) Silicon dioxide particles (%) | | | | | | | |
| | 0.05 | 0.01 | | | | 0.01 | 0.01 |
| (to mass of 1-methyl-phospholine-1-oxide) | (476) | (95) | | | | (95) | (95) |
| Specific surface area | 650 | 650 | | | | 300 | 350 |
| (m²/g) | 350 | 350 | | | | 105 | 160 |
| Oil absorption | | | | | | | |
| (mL/100 g) | | | | | | | |
| TMST (ppm) | | | 10 | | | | |
| Aluminum oxide (%) | | | | 0.05 | | | |
| NCO content (%) at 1 hour after addition of adsorbent or terminator | 29.6 | 29.4 | 29.3 | 29.4 | 29.5 | 29.1 | 29.3 |
| NCO content (%) 4 hours after addition | 29.4 | 29.1 | 28.9 | 29.0 | 28.7 | 28.5 | 28.8 |
| Filtration (minutes) | 50 | 9 | not needed | 5 | not needed | 3 | 96 |
| hardness in filtration | easy | easy | | easy | | easy | hard |
| NCO content (%) after filtration and 4 hrs or more after filtration | 29.4 | 29.0 | 28.9 | 28.7 | 28.5 | 27.7 | 28.7 |
| Viscosity (mPas/25°C)/ | 37/30 | 47/30 | 47/80 | 42/30 | 52/30 | 81/30 | 63/30 |
| Color number (APHA) | | | | | | | |
| (3) Storage stability (45°CX24hrs.) Expansion of 500-mL petroleum drum (levels 0 to 6) | 1 | 1 | 2 | 3 | 4 | 3 | 2 |

## Claims

1. A process for preparing a modified polyisocyanate by conducting a carbodiimidization reaction of an organic polyisocyanate in the presence of a phospholine-based catalyst, **characterized in that** silicon dioxide particles having a specific surface area (JIS K1150) of at least 400 m²/g and an oil absorption (JIS K5101) of at least 180 mL/100 g are used to adsorb and remove the phospholine-based catalyst, thereby terminating the carbodiimidization reaction.

2. The process according to claim 1, wherein the phospholine-based catalyst is used in an amount of 0.5 to 20 ppm based on the weight of the organic polyisocyanate.

3. The process according to claim 1 or 2, wherein the silicon dioxide particles are used in an amount of 2.5 to 2,000 wt. parts per 1 wt. part of the phospholine-based catalyst.

4. The process according to any one of claims 1 to 3, wherein the silicon dioxide particles have a specific surface area of 400 to 800 m²/g and an oil absorption of 180 to 500 ml/100 g.

5. A modified polyisocyanate obtained by the process according to any one of claims 1 to 4.

6. A modified polyisocyanate mixture comprising the modified polyisocyanate according to claim 5 and an organic polyisocyanate which is not carbodiimidized.

7. A prepolymer having a terminal isocyanate group, obtained from the modified polyisocyanate according to claim 5 or the modified polyisocyanate mixture according to claim 6.

## Patentansprüche

1. Verfahren zur Herstellung eines modifizierten Polyisocyanats durch Durchführung einer Carbodiimidierungsreaktion eines organischen Polyisocyanats in Gegenwart eines Katalysators auf Phospholin-Basis, **dadurch gekennzeichnet, daß** man Siliciumdioxidteilchen mit einer spezifischen Oberfläche (JIS K1150) von mindestens 400 m²/g und einer Ölabsorption (JIS K5101) von mindestens 180 mL/100 g zur Absorption und Entfernung des Katalysators auf Phospholin-Basis verwendet, wodurch die Carbodiimidierungsreaktion abgebrochen wird.

2. Verfahren nach Anspruch 1, bei man man den Katalysator auf Phospholin-Basis in einer Menge von 0,5 bis 20 ppm, bezogen auf das Gewicht des organischen Polyisocyanats, verwendet.

3. Verfahren nach Anspruch 1 oder 2, bei dem man die Siliciumdioxidteilchen in einer Menge von 2,5 bis 2000 Gewichtsteilen pro 1 Gewichtsteil des Katalysators auf Phospholin-Basis verwendet.

4. Verfahren nach einem der Ansprüche 1 bis 3, bei dem die Siliciumdioxidteilchen eine spezifische Oberfläche von 400 bis 800 m²/g und eine Ölabsorption von 180 bis 500 ml/100 g aufweisen.

5. Modifiziertes Polyisocyanat, erhalten nach dem Verfahren nach einem der Ansprüche 1 bis 4.

6. Modifiziertes Polyisocyanat enthaltende Mischung, umfassend das modifizierte Polyisocyanat nach Anspruch 5 und ein nicht carbodiimidisiertes organisches Polyisocyanat.

7. Prepolymer mit Isocyanat-Endgruppe, erhalten aus dem modifizierten Polyisocyanat nach Anspruch 5 oder der modifiziertes Polyisocyanat enthaltenden Mischung nach Anspruch 6.

## Revendications

1. Procédé de préparation d'un polyisocyanate modifié par la mise en oeuvre d'une réaction de carbodiimidation d'un polyisocyanate organique en présence d'un catalyseur à base de phospholine, **caractérisé en ce que** des particules de dioxyde de silicium ayant une surface spécifique (JIS K1150) d'au moins 400 m²/g et une absorption d'huile (JIS K5101) d'au moins 180 ml/100 g sont utilisées pour adsorber et enlever le catalyseur à base de phospholine, ce qui arrête de cette manière la réaction de carbodiimidation.

2. Procédé selon la revendication 1, dans lequel le catalyseur à base de phospholine est utilisé en une quantité de 0,5 à 20 ppm sur la base du poids du polyisocyanate organique.

3. Procédé selon la revendication 1 ou 2, dans lequel les particules de dioxyde de silicium sont utilisées en une quantité de 2,5 à 2 000 parties en poids pour 1 partie en poids du catalyseur à base de phospholine.

4. Procédé selon l'une quelconque des revendications 1 à 3, dans lequel les particules de dioxyde de silicium ont une surface spécifique de 400 à 800 m²/g et une absorption d'huile de 180 à 500 ml/100 g.

5. Polyisocyanate modifié obtenu par le procédé selon l'une quelconque des revendications 1 à 4.

6. Mélange de polyisocyanate modifié comprenant le polyisocyanate modifié selon la revendication 5 et un polyisocyanate organique qui n'est par carbodiimidé.

7. Prépolymère ayant un groupe isocyanate terminal, obtenu à partir du polyisocyanate modifié selon la revendication 5 ou du mélange de polyisocyanate modifié selon la revendication 6.
